# FASCICULE DE BREVET EUROPEEN

(11) **EP 0 931 252 B1**
(45) Date de publication et mention de la délivrance du brevet: **06.10.2004**
(21) Numéro de dépôt: 98942717.4
(22) Date de dépôt: 10.08.1998
(51) Int. Cl.: G01M 3/20

(54) **ANALYSEUR DE GAZ**
GASANALYSEGERÄT
GAS ANALYSING APPARATUS

(30) Priorité: 11.08.1997 FR 9710259
(43) Date de publication de la demande: 28.07.1999
(73) Titulaire: ALCATEL, 75382 Paris Cédex 08 (FR)
(72) Inventeur: DEVANCE, Alain, F-73400 Ugine (FR)
(74) Mandataire: Lamoureux, Bernard
(86) Numéro de dépôt international: PCT/FR1998/001782
(87) Numéro de publication internationale: WO 1999/008086

(56) Documents cités:
- FR-A- 2 366 553
- FR-A- 2 606 878
- FR-A- 2 688 307
- B. PI GAY: "Les détecteurs de fuites à hélium. Conception et étalonnage" LE VIDE: SCIENCE, TECHNIQUE ET APPLICATIONS., vol. 51, no. 278, novembre 1995, pages 443-455, XP000557206 FR
- J.C. LEGRAS, ET AL.: "Létalonnage des fuites de référence au BNM/L.N.E." LE VIDE: SCIENCE, TECHNIQUE ET APPLICATIONS., vol. 51, no. 278, novembre 1995, pages 436-442, XP000557205 FR

## Description

La présente invention concerne un analyseur de gaz comportant un groupe de pompage et des moyens d'autocalibration.

En particulier, l'invention s'applique à un analyseur de gaz faisant partie d'un détecteur de fuite à gaz traceur.

Les détecteurs de fuite à gaz traceur comportent généralement des moyens d'autocalibration c'est-à-dire d'étalonnage du détecteur. Pour cela, à l'intérieur de l'appareil, est situé un récipient de gaz traceur, en général de l'hélium, ayant un débit de fuite calibré et connu.

Ainsi, le document de B. PIEGAY « Les détecteurs de fuites à hélium - Conception et étalonnage » Le Vide : Science, Technique et Applications, vol. 51, n° 278, novembre 1995, pages 443-455, décrit différentes méthodes de contrôle d'étanchéité à l'hélium dont ladite fuite calibrée d'hélium.

Ce récipient est relié à l'entrée du détecteur par l'intermédiaire d'une vanne dont l'ouverture est commandée lors de l'opération d'autocalibration. Afin de permettre l'étalonnage, la valeur du flux de fuite calibrée est entrée dans un circuit électronique, par exemple au moyen de roues codeuses situées dans l'appareil, le circuit électronique agissant sur le réglage du courant de ionisation de l'analyseur de gaz. Par ailleurs sur le boîtier de l'appareil est prévu un commutateur de sélection de masse, généralement trois masses distinctes possibles, agissant sur la tension d'accélération des ions. Ceci bien que le détecteur ne contienne qu'un seul récipient de fuite calibrée pour un gaz déterminé.

Ainsi, si l'on veut utiliser un autre gaz traceur que celui contenu dans le récipient de fuite calibrée, situé à l'intérieur de l'appareil, il est nécessaire d'utiliser une bouteille à fuite calibrée de cet autre gaz et de la relier à la bride d'entrée de l'appareil et bien entendu d'ouvrir l'appareil pour afficher sur les roues codeuses la valeur de la fuite calibrée de cette bouteille. Il faut également placer le commutateur de sélection de masse sur la position correspondante au gaz utilisé.

Ceci étant effectué, lors de l'opération d'autocalibration, les paramètres ci-dessus : tension d'accélération des ions et courant d'ionisation sont réglés automatiquement de façon à ce que la fuite affichée sur l'appareil corresponde à la valeur du flux de fuite calibrée introduite sur les roues codeuses.

La présente invention a donc pour but de proposer un analyseur de gaz équipé de moyens permettant son étalonnage automatique pour une pluralité de gaz distincts, d'une façon très simple.

Un tel analyseur pouvant être utilisé directement pour l'analyse de gaz ou faire partie d'un détecteur de fuite utilisable avec plusieurs gaz traceurs distincts en simplifiant à l'extrême les manoeuvres à effectuer lors d'un changement du gaz traceur utilisé.

L'invention a ainsi pour objet un analyseur de gaz relié à un groupe de pompage et comportant des moyens d'autocalibration, caractérisé en ce qu'il est relié à un réservoir ayant une fuite calibrée, rempli d'un mélange de n gaz de masse distincte dont le flux de fuite de chacun à travers ladite fuite calibrée est connu.

Selon une réalisation avantageuse de l'invention, la concentration de chacun des n gaz du mélange est telle que ledit flux de fuite partiel de chacun des n gaz à travers ladite fuite calibrée est le même pour tous. Ceci permet d'afficher une valeur unique de flux de fuite, valable pour les n gaz.

On va maintenant donner la description d'un exemple de mise en oeuvre de l'invention en se reportant au dessin annexé dans lequel :
- la figure 1 est un schéma d'un détecteur de fuite à gaz traceur comportant un analyseur de gaz selon l'invention,
- la figure 2 est un schéma d'un analyseur de gaz selon l'invention utilisé directement pour l'analyse d'un flux de gaz.

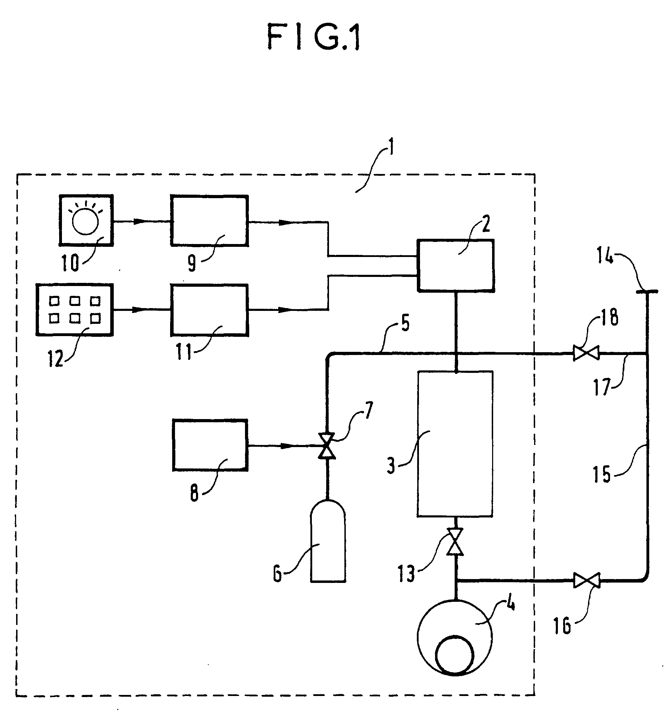

En se reportant à la figure 1, on voit un détecteur de fuite à gaz traceur utilisant un analyseur de gaz conforme à l'invention. L'analyseur de gaz, situé dans le cadre en trait discontinu repéré 1, comprend un spectromètre de masse 2 avec son groupe de pompage comprenant une pompe secondaire 3 et une pompe primaire 4, et des moyens d'autocalibration.

Les moyens d'autocalibration comprennent tout d'abord un réservoir 6 ayant une fuite calibrée. (par exemple un orifice calibré). Ce réservoir est relié au spectromètre 2 par une conduite 5 par l'intermédiaire d'une vanne 7 commandée par un circuit électronique d'automatisme 8.

Conformément à l'invention, le réservoir 6 est rempli d'un mélange de n gaz de masse distincte. (par exemple de l'Hélium 4, de l'Hélium 3 et de l'hydrogène). Le flux de fuite partiel de chacun des n gaz à travers la fuite calibrée est évidemment connu.

De préférence, la concentration de chacun des n gaz du mélange est choisie de façon à ce que son flux de fuite partiel soit identique à celui de chacun des autres gaz du mélange. Les moyens d'autocalibration comprennent en outre un circuit électronique 9 qui agit sur la tension d'accélération des ions formés dans le spectromètre de masse 1, pour la sélection de la masse. Ce circuit électronique est commandé par un bouton 10 de sélection de masse. Enfin, un circuit électronique 11 agit sur le courant électronique émis par la cathode (filament ou cathode froide) du spectromètre pour le réglage du courant de ionisation (réglage de la sensibilité). Les paramètres de ce circuit électronique sont réglés par des roues codeuses 12 sur lesquelles est affichée la valeur du flux de fuite partiel de chacun des gaz du réservoir 6 (une seule valeur puisque l'on a choisi une concentration telle pour chaque gaz que son flux de fuite partiel est le même que celui des autres gaz.

Pour être utilisé en détecteur de fuite, une vanne 13 est disposée entre la pompe primaire 4 et la pompe secondaire 3 et d'autre part une bride d'entrée 14, destinée à être reliée à une enceinte à tester, est reliée d'une part à la pompe primaire 4 par une conduite 15 équipée d'une vanne de prévidage 16 et d'autre part au spectromètre 2 par une conduite 17 équipée d'une vanne d'entrée 18.

Avec un tel appareil, lorsque l'on change de gaz traceur, il suffit, lors de l'étalonnage, d'afficher avec le bouton 10 la masse du gaz utilisé.

Si le flux de fuite de chaque gaz n'est pas le même, il faut en outre modifier la valeur du flux de fuite sur les roues codeuses 12. C'est pourquoi il est préférable de choisir pour chaque gaz du réservoir 6 une concentration telle que la fuite partielle de chaque gaz est la même pour tous.

Avec un tel appareil, un seul réservoir 6 est nécessaire.

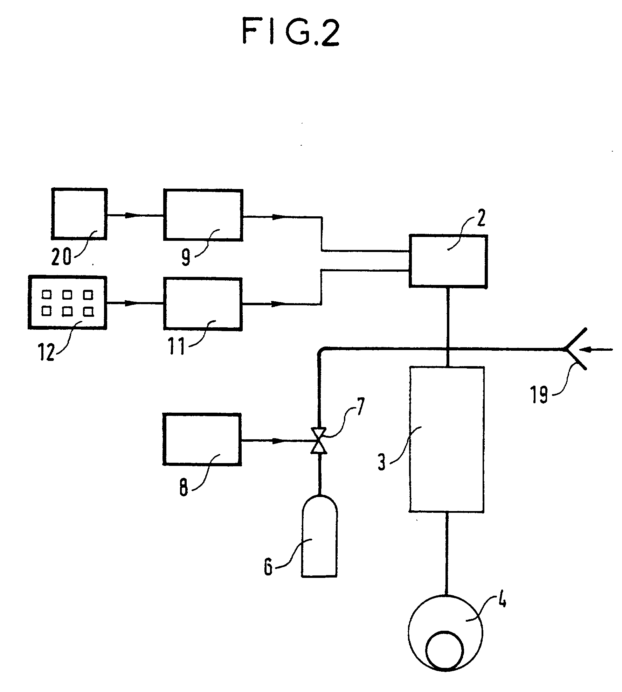

Dans le cas de la figure 2, l'analyseur de gaz est directement utilisé pour l'analyse, en spectrométrie de masse, d'un mélange gazeux à analyser. Ce mélange est introduit en 19.

Le réservoir 6 est donc ici utilisé pour étalonner l'analyseur pour chacun des n gaz qu'il contient grâce à la valeur de fuite connu de chacun des gaz : comme précédemment, on choisit de préférence une fuite partielle identique pour chaque gaz en choisissant les concentrations idoines. Dans cette application, le circuit électronique 9 comporte une mémoire et le bouton 10 est remplacé par un commutateur cyclique 20 balayant les différentes masses de gaz contenus dans le réservoir 6. Lors de l'étalonnage, la vanne 7 est ouverte, et le réglage de la tension d'accélération correspondant à chaque masse est mémorisé dans la mémoire du circuit 9. Lors de l'analyse d'un gaz envoyé en 19, vanne 7 fermée, le commutateur 20 passe en revue toutes les masses successivement et le circuit 9 règle pour chaque masse la valeur de la tension d'accélération mémorisée respectivement pour chaque masse lors de l'étalonnage.

## Revendications

1. Analyseur de gaz (1) comprenant un moyen d'analyse (2) avec son groupe de pompage (3, 4) et des moyens d'autocalibration (5 à 12), **caractérisé en ce qu'**il est relié à un réservoir (6) ayant une fuite calibrée, ledit réservoir étant rempli d'un mélange de n gaz de masse distincte dont le flux de fuite partiel de chacun à travers ladite fuite calibrée est connu, et ledit réservoir servant à étalonner ledit analyseur pour chacun des n gaz qu'il contient grâce à la valeur de fuite connue pour chacun des n gaz, ledit analyseur étant en outre **caractérisé en ce que** ledit moyen d'analyse (2), lesdits moyens d'autocalibration (5 à 12) et le groupe de pompage (3, 4) sont reliés à une entrée (14, 19) du gaz à analyser.

2. Analyseur de gaz selon la revendication 1 tel que la concentration de chacun des n gaz du mélange est telle que ledit flux de fuite partiel de chacun des n gaz à travers ladite fuite calibrée est le même pour tous.

3. Analyseur de gaz selon l'une des revendications 1 ou 2 tel qu'il fait partie d'un détecteur de fuite (1, 13 à 18).

## Patentansprüche

1. Gasanalysegerät (1), aufweisend eine Analysevorrichtung (2) mit ihrem Pumpenaggregat (3,4) und Selbstkalibrierungsvorrichtungen (5 bis 12), **dadurch gekennzeichnet, dass** es mit einem Behälter (6) mit einem kalibrierten Leck verbunden ist, wobei dieser Behälter mit einem Gemisch aus n Gasen unterschiedlicher Masse gefüllt ist, bei denen der partielle Leckstrom jedes Gases durch das kalibrierte Leck bekannt ist, und wobei der Behälter dazu dient, das Analysegerät für jedes der n Gase, welche er enthält, mit Hilfe des bekannten Leckwertes für jedes der n Gase zu eichen; hierbei ist das Analysegerät außerdem **dadurch gekennzeichnet, dass** die Analysevorrichtung (2), die Selbstkalibrierungsvorrichtungen (5 bis 12) und das Pumpenaggregat (3, 4) mit einem Einlass (14, 19) des zu analysierenden Gases verbunden sind.

2. Gasanalysegerät nach Anspruch 1, bei dem die Konzentration jedes der n Gase des Gemischs so ist, dass der partielle Leckstrom jedes der n Gase durch das kalibrierte Leck für alle Gase derselbe ist.

3. Gasanalysegerät nach einem der Ansprüche 1 und 2, bei dem dieses Gasanalysegerät zu einem Lecksucher (1, 13 bis 18) gehört.

## Claims

1. Gas analyser (1) comprising an analysis means (2) with its pump unit (3, 4) and autocalibration means (5 to 12), **characterized in that** it is connected to a storage tank (6) having a calibrated leak, said storage tank being filled with a mixture of n gases having different masses and the partial leakage flow of each of which through said calibrated leak is known and said storage tank being used to calibrate said analyser for each of the n gases that it contains using the known leakage value for each of the n gases, said analyser being further **characterized in that** said analysis means (2), said auto calibration means (5 to 12), and the pump unit (3, 4) are connected to an inlet (14, 19) for the gas to be analysed.

2. Gas analyser according to claim 1 such that the concentration of each of the n gases in the mixture is such that said partial leakage flow of each of the n gases through said calibrated leak is the same for all of them.

3. Gas analyser according to claim 1 or claim 2 such that it is part of a leak detector (1, 13 to 18).
